Europäisches Patentamt

European Patent Office

Office européen des brevets

(11) Veröffentlichungsnummer: **0 299 240**

**A2**

# (12) EUROPÄISCHE PATENTANMELDUNG

(21) Anmeldenummer: 88109909.7

(51) Int. Cl.⁴: **H04N 5/445**

(22) Anmeldetag: **22.06.88**

(30) Priorität: **14.07.87 DE 3723228**

(43) Veröffentlichungstag der Anmeldung:
**18.01.89 Patentblatt 89/03**

(84) Benannte Vertragsstaaten:
**AT BE DE FR GB NL**

(71) Anmelder: **Richard Wolf GmbH**
**Pforzheimer Strasse 32**
**D-7134 Knittlingen(DE)**

(72) Erfinder: **Ams, Felix, Dipl.-Ing.**
**Gründle Strasse 5**
**D-7539 Kämpfelbach(DE)**
Erfinder: **Schäfer, Roland**
**Bannzaunstrasse 13**
**D-7518 Bretten-Diedelsheim(DE)**

(74) Vertreter: **Wilcken, Hugo, Dr. et al**
**Patentanwälte Dr. Hugo Wilcken Dipl.-Ing.**
**Thomas Wilcken Musterbahn 1**
**D-2400 Lübeck(DE)**

(54) **Endoskopische Fernsehanlage.**

(57) Es ist eine endoskopische Fernsehanlage (10) beschrieben, bei der das von einer Fernsehkamera (11) über eine Optik des Endoskopes oder von einem im Endoskop integrierten Bildwandler aufgenommene Bild des jeweiligen Objektes als Videosignal einem Monitor (13) zugeführt unter Freilassen von bildfreien Randzonen auf dem Bildschirm des Monitors abgebildet wird. Die endoskopische Fersehanlage (10) weist dazu einen Zeichengenerator (14) auf, der über eine Tastatur (15) ansteuerbar ist, um durch Betätigung der Tastatur ausgewählte Zeichen in einem Zeichenspeicher (25) abzuspeichern. Eine Bildsteuerung wird mit aus dem Videosignal abgeleiteten Synchronisierungsimpulsen betrieben, um die abgespeicherten Zeichen aus dem Zeichenspeicher auszulesen und dem einen Eingang (19) eines Mischers (18) zuzuführen, dessen anderem Eingang (20) das Videosignal zugeführt wird. Der Ausgang (21) des Mischers (18), der das aus dem Videosignal und dem ausgelesenen Zeichensignal gemischte Bild-Zeichen-Signal führt, ist mit dem Monitor (13) verbunden und der Zeichengenerator (14) ist so programmiert, daß die Zeichensignale in wenigstens einer der vorerwähnten Randzonen auf dem Monitor (13) abgebildet werden.

Fig. 1

## Endoskopische Fernsehanlage

Die Erfindung bezieht sich auf eine endoskopische Fernsehanlage, bei der das von einer Fernsehkamera über eine Optik des Endoskopes oder von einem im Endoskop integrierten Bildwandler aufgenommene Bild des jeweiligen Objektes als Videobild einem Monitor zugeführt und unter Freilassen von bildfreien Randzonen auf dem Bildschirm des Monitors abgebildet wird.

Endoskopische Fernsehanlagen dieser Art sind bekannt (DE-PS 34 36 057, EP-PS 0 076 937), bei denen entweder von einer Fernsehkamera oder einem Halbleiter-Bildwandler (CCD-Bildwandler) aufgenommene Bilder, die von den damit verbundenen Endoskopen aufgenommen worden sind, auf Monitore oder sonstige geeignete Anzeigegeräte gegeben werden. Alle diese bekannten endoskopischen Fernsehanlagen liefern Bildinformationen, die auf dem Monitor im wesentlichen in Form eines kreisrunden Bildes erscheinen, während die übrigen Randbereiche eines im wesentlichen rechteckförmigen Monitorbildschirms keine Bildinformation zeigen.

Während einer endoskopischen Beobachtung müssen bestimmte patientenspezifische Daten, wie Namen, Alter, Uhrzeit der Beobachtung und dgl., festgehalten werden, d. h. in eine bestimmte Korrelation zum dargestellten Bild gebracht werden. Auch sind bestimmte gerätespezifische Beobachtungsparameter dem Videobild zuzuordnen und auch bestimte Parameter von externen Geräten zur Behandlung des Patienten, wenn beispielsweise mit der endoskopischen Beobachtung ein operativer oder therapeutischer Eingriff einhergeht. Die vorbeschriebenen gesamten Parameter, d. h. die für eine mit der endoskopischen Beobachtung einhergehende Dokumentation notwendigen Informationen wurden bisher entweder auf einem gesonderten Aufzeichnungsträger aufgezeichnet und nach der Beobachtung in eine bestimmte Korrelation zum Videobild gebracht, oder eine entsprechende Dokumentation wurde in Form eines gesonderten Teilbildes, beispielsweise in Form einer rechteckigen Einblendung in das Videobild, eingefügt.

Beide Vorgehensweisen sind insofern nachteilig, als einerseits bei Eingabe in einen gesonderten Datenträger nachträglich eine bestimmte Korrelation zum Videobild durchgeführt werden muß, was sehr zeitaufwendig ist und darüber hinaus auch einen erheblichen apparativen und manuellen Arbeitsaufwand erfordert, und andererseits wird die Einblendung eines Teilfernsehbildes das Vorhandensein eines gesonderten Trickmischpultes erfordern, wobei zusätzlich zum Trickmischpult auch noch eine gesonderte Eingabeeinrichtung bzw. eine Wandlereinrichtung nötig ist, um die einzugebende Dokumentation in Videosignale umzuwandeln und über das Trickmischpult in Form eines Teilbildes in das Gesamtbild einzublenden.

Es ist Aufgabe der vorliegenden Erfindung, eine endoskopische Fernsehanlage zu schaffen, mit der die videosignalfreien Randzonenbereiche eines grundsätzlich im wesentlichen rechteckigen Bildes auf dem Bildschirm eines Monitors mit Informationen mit einfachen Mitteln angefüllt werden können, die im Zusammenhang mit der endoskopischen Beobachtung und ggf. einhergehenden chirurgischen oder therapeutischen Behandlungen entstehen, so daß gesonderte zusätzliche Einrichtungen zur Darstellung der Dokumentation neben dem eigentlichen Videobild auf dem Monitorschirm nicht erforderlich sind.

Gelöst wird die Aufgabe gemäß der Erfindung dadurch, daß ein Zeichengenerator mit einer Tastatur ansteuerbar ist, um durch Betätigung der Tastatur ausgewählte Zeichen in einem Zeichenspeicher abzuspeichern, daß eine Bildsteuerung mit aus dem Videosignal abgeleiteten Synchronisierungsimpulsen betrieben wird, um die abgespeicherten Zeichen aus dem Zeichenspeicher auszulesen und dem einen Eingang eines Mischers zuzuführen, dessen anderem Eingang das Videosignal zugeführt wird, daß der Ausgang des Mischers, der das aus dem Videosignal und dem ausgelesenen Zeichensignal gemischte Bild-Zeichen-Signal führt, mit dem Monitor verbunden ist und daß der Zeichengenerator so programmiert ist, daß die Zeichensignale in wenigstens einer der vorerwähnten Randzonen auf dem Monitor abgebildet werden.

Der Vorteil einer derartigen Fernsehanlage besteht darin, daß gesonderte Einrichtungen wie ein Trickmischpult und zusätzliche Signal-Bild-Wandler nicht nötig sind und gemäß der Erfindung zwei oder mehrere voneinander getrennte Bild- bzw. Speicherbereiche in den bildfreien Randzonen des Monitorbildes möglich sind. Darüber hinaus kann durch unterschiedliche Programmsteuerung die Art der einzugebenden und anzuzeigenden Parameter in den bildfreien Randzonen sehr variabel gestaltet werden, da dazu lediglich Softwareänderungen im Steuerprogramm nötig sind. Die gesamte auf den bildfreien Randzonen darstellbare Dokumentation ist zu jedem Augenblick richtig zum dargestellten Videobild, das vom Endoskop geliefert wird, korreliert.

Gemäß einer vorteilhaften Ausgestaltung der Fernsehanlage wandelt die Bildsteuerung von einem Mikroprozessor gesteuert den Tastencode der von der Tastatur ankommenden Signale in fernsehkompatible Zeichensignale um und plaziert diese im Zeichenspeicher in Abhängigkeit von einem in einem Programmspeicher befindlichen Programm derart, daß die auszulesenden Zeichensignale in wenigstens einer der Randzonen zur Abbildung kommen. Grundsätzlich ist es so, daß die Bildsteuerung von einer beliebigen, entweder fest programmierten oder rechnergestützten Steuerung gesteuert werden können, beispielsweise auch durch einen externen Rechner, der mit der Bildsteuerung zusammen arbeitet. Es hat sich jedoch herausgestellt, daß durch Vorsehen eines Mikroprozessors in der Fernsehanlage die Anpaßbarkeit an momentane Beobachtungsvorgaben oder einhergehende chirurgische oder therapeutische Maßnahmen vergrößert wird, da einerseits das Programm den entsprechenden Beobachtungsvorgaben schnell angepaßt werden kann und andererseits die Steuerung durch einen externen Rechner einer flexiblen Einsatzmöglichkeit der Fernsehanlage zuweilen entgegensteht.

Gemäß einer anderen vorteilhaften Ausgestaltung der Fernsehanlage sind in den Zeichengenerator extern erzeugte und im Zeichenspeicher einschreibbare sowie zur Anzeige auf den Monitor auslesbare Meß- und/oder Betriebsparameter eingebbar. Dafür weist die Fernsehanlage einen oder mehrere gesonderte Eingänge auf, so daß beispielsweise bestimmte Parameter von Geräten, die während der Operation oder der Therapie bestimmte Daten liefern, eingeblendet werden können. Darüber hinaus können diese zusätzlich eingeblendeten Meß- und Betriebsparameter auch von der Fernsehanlage selbst herrühren, um beispielsweise während der Beobachtung fortwährend über den ordnungsgemäßen Betriebs- bzw. Funktionszustand der Fernsehanlage unterrichtet zu sein.

Vorzugsweise können die eingebbaren Meß- und/oder Betriebsparameter in für einen vorbestimmten endoskopischen oder chirurgischen Eingriff erforderlichen Hilfseinrichtungen erzeugt werden, beispielsweise durch Anästhesie, Analgesie oder chirurgische Einrichtungen, um fortwährend eine exakte Dokumentation bei der Beobachtung zu haben, so daß die derartige Untersuchungen und Eingriffe durchführenden Personen mit einem Blick sämtliche wesentlichen Parameter erfassen.

Vorteilhafterweise wird von der Fernsehanlage ein akustisches Signal erzeugt, wenn die Grenzen innerhalb vorstellbarer Grenzen einstellbarer Meß- und/oder Betriebsparameter überschritten werden, so daß beispielsweise bei Beobachtungen, die die ganze Aufmerksamkeit der Bedienungsperson bei der Beobachtung des endoskopischen Bildes erfordern, ein Überschreiten bzw. ein Unterschreiten vorbestimmter Werte nicht unbemerkt bleibt.

Genau das gleiche Ergebnis kann vorzugsweise dadurch erzielt werden, daß die innerhalb vorgebbarer Grenzen einstellbaren und auf dem Monitor anzeigbaren Meß- und/oder Regelparameter optisch hervorgehoben werden, wenn die Grenzen überschritten werden, beispielsweise durch eine alternierende Hell-Dunkel-Tastung der angezeigten Parameter oder eine gegenüber der normalen Darstellung zyklische Hellertastung der Parameter.

Schließlich ist es von Vorteil, wenn bei der Fernsehanlage das entsprechend der Abbildung auf dem Monitor aus Endoskopbild (Videosignalfolge) und Zeichenbild erzeugte Gesamtbild durch eine Bildspeichereinrichtung gespeichert wird, so daß entweder kontinuierlich oder in bestimmten zeitlichen Abständen eine genaue Wiedergabe des gesamten Monitorbildes möglich ist und auch im Nachhinein eine eindeutige Korrelation zwischen Endoskopbild und dem Zeichenbild existiert.

Die Erfindung wird nachfolgend unter Bezugnahme auf die schematischen Zeichnungen anhand eines Ausführungsbeispieles eingehend erläutert. Darin zeigen:

Figur 1 ein Blockschaltbild der endoskopischen Fernsehanlage,

Figur 2 das Blockschaltbild eines Zeichengenerators als Teil der in Figur 1 dargestellten Fernsehanlage und

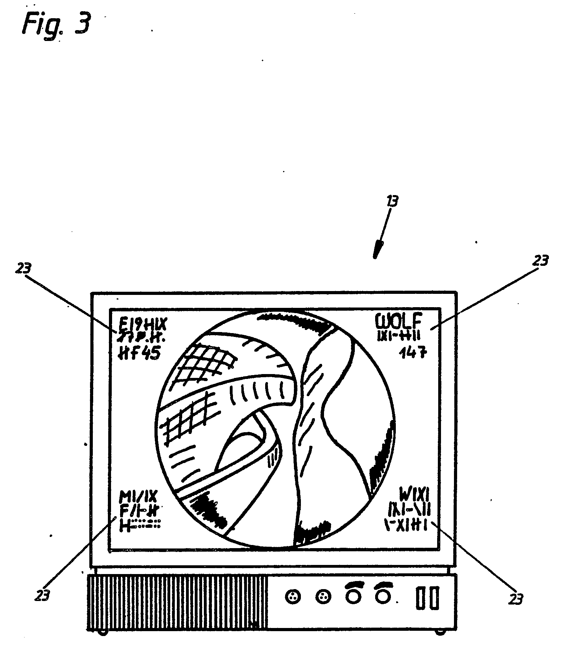

Figur 3 einen Monitorbildschirm, auf dem das Endoskopbild in der Mitte des Bildschirms und in den bildfreien Randzonen Zeichenbilder dargestellt sind.

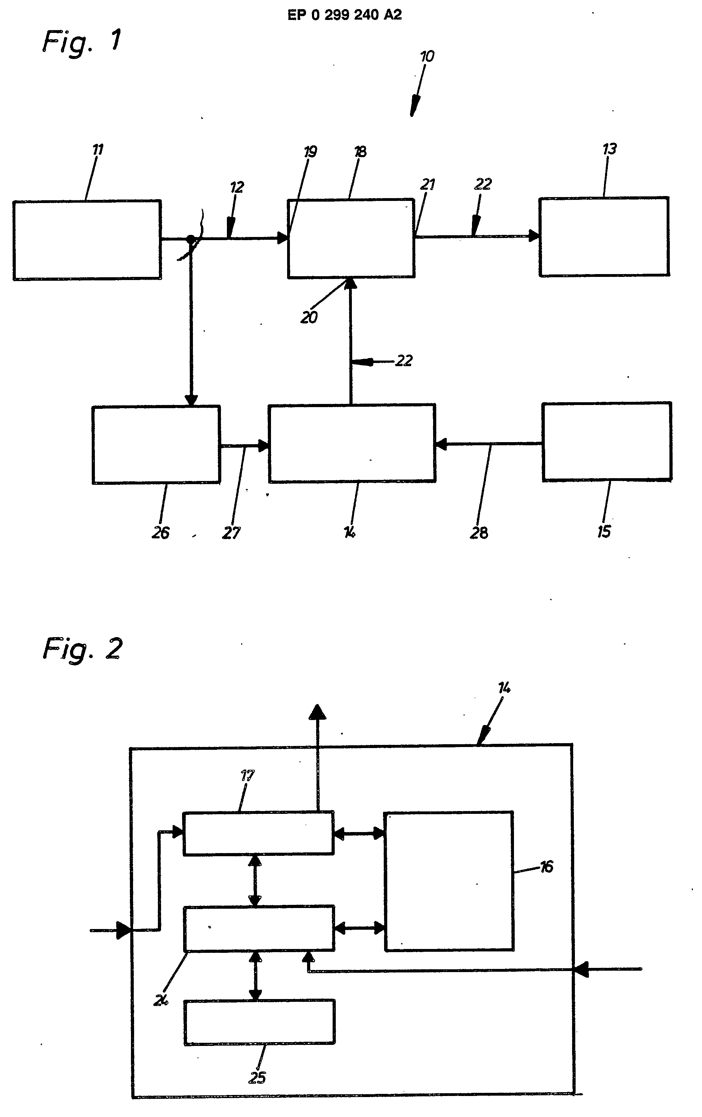

Die endoskopische Fernsehanlage 10 besteht, wie in Figur 1 dargestellt, im wesentlichen aus einer Fernsehkamera 11, die entweder aus einem konventionell aufgebauten Videosignalaufnahmesystem oder aus einem Halbleiter-Bildwandler (CCD-Bildwandler) bestehen kann und aus einem Monitor 13, auf den bei den bisherigen Fernsehanlagen das von der Fernsehkamera 11 erzeugte Videosignal 12 gegeben wird. Bei der hier beschriebenen Fernsehanlage 10 ist zwischen dem Monitor 13 und der Fernsehanlage 10 ein Mischer 18 geschaltet, so daß das von der Fernsehkamera 11 gelieferte Videosignal 12 auf den einen Eingang 19 des Mischers 18 gegeben wird, während der Ausgang 21 des Mischers 18 das aus dem Zeichensignal 22 und dem Videosignal 12 gemischte Bild in Form eines Gesamtsignals 220 auf den Monitor 13 liefert, was im einzelnen noch weiter unten beschrieben wird.

Das von der Fernsehkamera 11 gelieferte Videosignal 12 wird ebenfalls auf eine Synchronimpulsabtrenneinrichtung 26 gegeben, deren Ausgang Synchronimpulse 27 an den Zeichengenerator 14 liefert, dessen Aufbau und Funktion im einzelnen im Zusammenhang mit der Beschreibung von Figur 2 weiter unten beschrieben wird.

Von einer Tastatur 15 wird ein dem Tastencode entsprechend der Betätigung der Taste geliefertes Tastensignal auf den Zeichengenerator 14 gegeben. Der Ausgang des Zeichengenerators 14 liefert Zeichensignale 22, die auf den zweiten Eingang 20 des Mischers gegeben werden.

In Funktion wird von der Fernsehkamera 11 eine auf bekannte Weise ausgebildete Folge von Videosignalen 12 geliefert, wobei in der Synchronimpulsabtrenneinrichtung 26 aus dem Videosignal 12 Synchronimpulse 27 abgetrennt werden, die dem Zeichengenerator 14 zugeführt werden. Mittels der Tastatur 15 können beispielsweise bestimmte auf dem Monitor 13 anzuzeigende Zeichen eingegeben werden, wobei die Tastatur 15 eine beliebige alphanumerische Tastatur sein kann. Entsprechend der Betätigung der Tasten der Tastatur 15 werden Tastensignale 28 auf den Zeichengenerator gegeben, und zwar bei dem hier dargestellten Ausführungsbeispiel auf den Mikroprezessor 24, vgl. Figur 2. Der Mikroprozessor 24 wird durch ein Softwareprogramm gesteuert, das beispielsweise dort in Form eines PROMs gespeichert wird. Ein derartiger Programmspeicher 25 in Form eines PROMs kann leicht ausgewechselt werden, d. h. unterschiedliche Programme zur Art der Darstellung der auf dem Monitor in den Randzonen 13 sichtbar zu machenden Zeichen können den momentan gewünschten Bedingungen leicht angepaßt werden. Der Mikroprozessor 24 steht mit einer Bildsteuerung 17 in Verbindung, wobei auf die Bildsteuerung 17 unmittelbar die Synchronimpulse 27 von der Synchronimpulsabtrenneinrichtung 26 gegeben werden. Der Mikroprozessor 24 steht ebenfalls mit einem Zeichenspeicher in Verbindung, ebenfalls wie die Bildsteuerung 17. Am Ausgang der Bildsteuerung 17 liegen die Zeichensignale 22, die, wie schon erwähnt, auf den zweiten Eingang 20 des Mischers 18 gegeben werden.

Die von der Tastatur 15 kommenden Tastensignale werden vom Mikroprozessor entsprechend dem im Programmspeicher 25 gespeicherten Programm plaziert. Von der Bildsteuerung 17 werden die von der Tastatur 15 kommenden Signale 28 in fernsehkompatible Zeichensignale 22 umgewandelt und im Zeichenspeicher 16 abgelegt. Aus dem Zeichenspeicher 16 werden dann die fernsehkompatiblen Zeichensignale ausgelesen und auf den Mischer 18 gegeben, und zwar geschieht das Auslesen entsprechend der Synchronsignale 27 in bildsignalsynchroner Form.

Im Mischer 18, der das Videosignal 12 mit dem videosignalsynchronen Zeichensignal 22 mischt, wird, wie schon erwähnt, eine aus beiden Signalen bestehendes Gesamtsignal 220 erzeugt, wobei die Zeichen auf dem Monitor 13 in den bildfreien Randzohonen 23 angezeigt werden.

Der Zeichengenerator 14 verhindert über das im Programmspeicher 25 gespeicherte Programm, daß die Bereiche der im wesentlichen kreisförmigen Abbildung des vom Endoskop herrührenden Videobildes von den Zeichen beschrieben werden, die mittels der Tastatur 15 eingegeben worden sind. D. h. mit anderen Worten, daß mittels der Tastatur 15 nur die bildfreien Randzonen 23 des auf dem Monitor 13 erscheinenden Bildes abgebildet werden können, was gemäß der Erfindung ausschließlich durch die geeignet gestaltete Programmsteuerung des in Programmspeicher 25 gespeicherten Programms erfolgt.

Obwohl hier nicht gesondert dargestellt, kann der Zeichengenerator noch mit zusätzlichen Eingängen versehen sein, über den die extern erzeugten Meß-und/oder Betriebsparameter eingebbar sind, die beispielsweise auf gleiche Weise wie die Tastensignale 28 in den Mikroprozessor 24 eingegeben und auf gleiche Weise wie die Tastensignale 28 von dem Zeichengenerator 14 verarbeitet werden können.

Bei diesen externen Betriebsparametern kann es sich um fernsehanlageneigne Meß- und/oder Betriebspara meter oder aber um solche handeln, die von Einrichtungen erzeugt werden, die neben der Fernsehanlage bei der endoskopischen Beobachtung benutzt werden, wobei es sich beispielsweise um von chirurgischen oder therapeutischen Einrichtungen herrührende Meß- und/oder Betriebsparameter handeln kann, die ebenfalls für die Gesamtanzeige auf dem Monitor 13 in Korrelation zum endoskopischen Bild nötig sind.

Darüber hinaus kann die Fernsehanlage auch mit einer hier nicht gesondert dargestellten Einrichtung versehen sein, die das Gesamtsignal 220, wie es zum Monitor 13 geliefert wird, aufzeichnet.

Außerdem kann auch noch vorgesehen sein, daß mittels hier ebenfalls nicht dargestellter Einrichtungen ein akustisches Signal ertönt, wenn die Grenzen innerhalb vorgebbarer Grenzen einstellbarer Meß- und/oder Betriebsparameter, die, wie schon erwähnt, ebenfalls auf dem Monitor 13 in den Randzonen 23 angezeigt werden können, überschritten worden sind. Auch ist es möglich, derartige Überschreitungen dadurch sichtbar zu machen, daß die entsprechende sichtbare Information auf dem Monitor 13 durch Hell-Dunkel-Tastung oder auf beliebige andere geeignete Weise gegenüber den dort dargestellten anderen Information sichtbar gemacht wird.

Weiterhin kann die Programmierung des Zeichengenerators 14 und in Verbindung hiermit die Auswahl der Randzonen 23 in Abhängigkeit von der Tastatur 15 verändert werden.

Auch besteht die Möglichkeit, das Endoskopbild auf dem Monitor in einer beliebigen und frei wählbaren Lage, Größe und Bildgeometrie abzubilden und den Bildschirm vollständig zur Darstellung des endoskopischen Bildes aus zunutzen, wobei dann frei wählbare Zeichen, Daten und Textangaben in das Bild eingeblendet werden können. Schließlich besteht auch die Möglichkeit, zur Darstellung von zeitlich abhängigen und aufeinanderfolgenden Abläufen mehrere ausgewählte Endoskopbilder abzuspeichern und diese einzeln oder gemeinsam in das aktuelle Endoskopbild einzublenden.

**Ansprüche**

1. Endoskopische Fernsehanlage, bei der das von einer Fernsehkamera über eine Optik des Endoskopes oder von einem im Endoskop integrierten Bildwandler aufgenommene Bild des jeweiligen Objektes als Videosignal einem Monitor zugeführt und unter Freilassen von bildfreien Randzonen auf dem Bildschirm des Monitors abgebildet wird, dadurch gekennzeichnet, daß ein Zeichengenerator (14) mit einer Tastatur (15) ansteuerbar ist, um durch Betätigung der Tastatur (15) ausgewählte Zeichen in einem Zeichenspeicher (16) abzuspeichern, daß eine Bildsteuerung (17) mit aus dem Videosignal abgeleiteten Synchronisierungsimpulsen betrieben wird, um die abgespeicherten Zeichen aus dem Zeichenspeicher (16) auszulesen und dem einen Eingang (20) eines Mischers (18) zuzuführen, dessen anderem Eingang (19) das Videosignal zugeführt wird, daß der Ausgang (21) des Mischers (18), der das aus dem Videosignal (12) und dem ausgelesenen Zeichensignal (22) gemischte Bild-Zeichen-Signal führt, mit dem Monitor (13) verbunden ist und daß der Zeichengenerator (14) so programmiert ist, daß die Zeichensignale (22) in wenigstens einer der vorerwähnten Randzonen (23) auf dem Monitor (13) abgebildet werden.

2. Fernsehanlage nach Anspruch 1, dadurch gekennzeichnet, daß die Bildsteuerung (17) von einem Mikroprozessor (24) gesteuert den Tastencode der von der Tastatur (15) ankommenden Signale in fernsehkompatible Zeichensignale (22) umwandelt und diese im Zeichenspeicher (16) in Abhängigkeit von einem in einem Programmspeicher (25) befindlichen Programm so plaziert, daß die auszulesenden Zeichensignale (22) in wenigstens einer der Randzonen (23) zur Abbildung kommen.

3. Fernsehanlage nach Anspruch 1 oder 2, dadurch gekennzeichnet, daß in den Zeichengenerator (14) extern erzeugte und in den Zeichenspeicher (16) einschreibbare sowie zur Anzeige auf den Monitor (13) auslesbare Meß- und/oder Betriebsparameter eingebbar sind.

4. Fernsehanlage nach Anspruch 3, dadurch gekennzeichnet, daß die eingebbaren Meß- und/oder Betriebsparameter in für einen vorbestimmten endoskopischen oder chirurgischen Eingriff erforderlichen Hilfseinrichtungen erzeugt werden.

5. Fernsehanlage nach Anspruch 3 oder 4, dadurch gekennzeichnet, daß von dieser ein akustisches Signal erzeugt wird, wenn die Grenzen innerhalb vorgebbarer Grenzen einstellbarer Meß- und/oder Betriebsparameter überschritten werden.

6. Fernsehanlage nach Anspruch 3 bis 5, dadurch gekennzeichnet, daß die innerhalb vorgebbarer Grenzen einstellbaren und auf dem Monitor (13) anzeigbaren Meß- und/oder Regelparameter optisch hervorhebbar sind, wenn die Grenzen überschritten werden.

7. Fernsehanlage nach Anspruch 1 bis 6, dadurch gekennzeichnet, daß das entsprechend der Abbildung auf dem Monitor (13) aus dem Endoskopbild (Videosignalfolge) und Zeichenbild erzeugte Gesamtbild durch eine Bildspeichereinrichtung speicherbar ist.

8. Fernsehanlage nach einem der Ansprüche 1 bis 7, dadurch gekennzeichnet, daß zur Darstellung zeitabhängiger Abläufe mehrere ausgewählte und einzeln abgespeicherte Endoskopbilder einzeln oder gemeinsam in das aktuelle Endoskopbild einblendbar sind.

9. Fernsehanlage nach einem der Ansprüche 1 bis 8, dadurch gekennzeichnet, daß die Programmierung des Zeichengenerators (14) und damit gekoppelt die Auswahl der Randzonen (23) in Abhängigkeit von der Betätigung der Tastatur (15) veränderbar ist.

**Fig. 1**

**Fig. 2**

# Fig. 3